# EUROPÄISCHE PATENTSCHRIFT

(11) **EP 3 324 859 B1**
(45) Veröffentlichungstag und Bekanntmachung des Hinweises auf die Patenterteilung: **23.03.2022**
(21) Anmeldenummer: 16734715.2
(22) Anmeldetag: 06.07.2016
(51) Int. Cl.: A61B 17/16, A61B 17/00, B25B 13/46, B25B 21/00, B25B 23/14, B25F 3/00, B25F 5/02

(54) **WERKZEUGAUFNAHMEAUFSATZ FÜR CHIRURGISCHE BOHRMASCHINE MIT ZUSÄTZLICHER MANUELLER ANTRIEBSEINHEIT UND CHIRURGISCHE BOHRMASCHINE**
SPINDLE FOR SURGICAL DRILL WITH ADDITIONAL MANUAL DRIVE AND SURGICAL DRILL
MANDRIN POUR PERCEUSE CHIRURGICALE AVEC UN ENTRAÎNEMENT SUPPLEMENTAIRE MANUEL ET PERCEUSE CHIRURGICALE

(30) Priorität: 22.07.2015 DE 102015111877; 22.07.2015 DE 102015111878
(43) Veröffentlichungstag der Anmeldung: 30.05.2018
(73) Patentinhaber: Aesculap AG, 78532 Tuttlingen (DE)
(72) Erfinder: HÖGERLE, Roland-Alois, 78532 Tuttlingen (DE); STEINHAUSER, Jan, 72488 Sigmaringen (DE)
(74) Vertreter: Winter, Brandl - Partnerschaft mbB
(86) Internationale Anmeldenummer: PCT/EP2016/065988
(87) Internationale Veröffentlichungsnummer: WO 2017/012877

(56) Entgegenhaltungen:
- DE-A1- 19 942 292
- DE-A1-102006 057 283
- DE-A1-102007 048 928
- DE-A1-102011 088 252
- DE-B4-102011 088 252
- DE-C2- 19 942 292
- US-A- 5 794 715

## Beschreibung

Die Erfindung betrifft einen Werkzeugaufnahmeaufsatz für eine medizinische, motorisch angetriebene Werkzeugmaschine wie beispielsweise chirurgische Bohrmaschine, die auch als chirurgischer Schraubendreher bezeichnet werden kann oder eingesetzt werden kann, mit einer antriebseitigen Kupplung zum Anbringen an einem (elektro-) motorisch Drehmoment bereitstellenden Antriebsaggregat/Motor, wie einer Antriebsmaschine mit einem Elektromotor und mit einer abtriebsseitigen Kupplung/Werkzeugaufnahmefutter zum Aufnehmen eines Werkzeugs, wie eines Schraubendrehers, eines Bohrers oder dergleichen Drehwerkzeuge.

In der Medizintechnik, insbesondere in der Chirurgie, werden bei bestimmten chirurgischen Eingriffen Knochenschrauben gesetzt, bspw. sogenannte Pedikelschrauben, welche in der Wirbelsäulenchirurgie Verwendung finden. Dazu muss erst ein Bohrvorgang durchgeführt werden, um danach in das in den Wirbelkörper gebohrte Loch jeweils eine Schraube setzen zu können. Leider sind die zu behandelnden Knochen/Wirbel immer unterschiedlich spröde. Man bedarf daher eines gewissen Feingefühls, um die Bohrung sauber zu setzen und die Schraube zerstörungsfrei/d.h. ohne Beschädigung des Wirbelkörpers einzubringen. Allerdings sind besonders die letzte Phase des Bohrvorganges und die letzte Phase des Schraubvorganges kritisch. Bis dahin bedarf es viel Drehbewegung, um das Bohrloch zu kreieren und die Schraube tief genug einzubringen, sodass das Risiko eines Ausreißens des Wirbelknochens oder Überdrehens der Schraube insbesondere in der jeweiligen Endphase des Bohr- und Schraubvorgangs ansteigt.

Ein manuelles Eindrehen von chirurgischen Schrauben führt jedoch durch die repetitive Bewegung zu einer schnellen Ermüdung der Hand- und Armmuskulatur des Chirurgen. Das kann dann zu einem negativen Operationsergebnis führen und auch langfristige gesundheitliche Beschwerden bei den Ärzten hervorrufen.

Es ist daher schon immer wünschenswert, ein motorisches Antriebsaggregat (elektrischer, hydraulischer oder pneumatischer Motor) zu nutzen, um Drehmoment bereit zu stellen. Dabei werden häufig elektromotorische Antriebsaggregate verwendet, also Antriebsmaschinen mit einem Elektromotor, die entweder batterie- / akkumulatorabhängig mit Energie versorgt werden, oder aber über ein Kabel auf eine externe Stromversorgung zugreifen.

Es sind bereits sogenannte Spickdrahtfutter bekannt, die Aufsätze für chirurgische Bohrmaschinen sind, welche einen Hebel zur Erfüllung einer speziellen Funktion verwenden. Durch die Maschine wird ein Spickdraht geführt. Wird der Hebel gezogen, wird der Draht in der Maschine geklemmt, sodass er durch die Antriebsmaschine in eine Rotationsbewegung versetzt werden kann. Dadurch kann der Draht in ein Gewebe eingedreht werden. Beim Loslassen des Hebels kann der Draht wieder in der Maschine axial und rotatorisch frei bewegt werden. Dieses Prinzip wird auch in anderen medizinischen Einrichtungen weiter verfolgt.

Benachbarter Stand der Technik ist bspw. aus der DE 10 2011 088 252 A1 bekannt. Dort wird ein Ratschenaufsatz zum Antrieb von Schraubwerkzeugen offenbart, die zum Lösen und Festziehen von Schraubelementen dienen und die wahlweise in beiden Drehrichtungen antreibbar sind, wobei der Ratschenaufsatz sowohl als geradliniger Aufsatz als auch als Winkelaufsatz ausführbar ist. In einem hülsenförmigen Gehäuse sind zwei im Abstand voneinander angeordnete, relativ zu dem Gehäuse drehbar aber axial feststehend gelagerte Mitnehmer vorgesehen. Die Mitnehmer weisen an ihren einander zugewandten Stirnseiten in ihrem Aufwandsbereich je eine Ratschenverzahnung auf, die gegenläufig zueinander ausgebildet sind. Gegenüber der jeweiligen Ratschenverzahnung der beiden Mitnehmer ist ein Antriebsring vorgesehen, der auf der dem jeweiligen Mitnehmer zugewandten Seite mit einer entsprechenden, mit diesem koppelbaren, Ratschenverzahnung versehen ist. Der Antriebsring ist in dem Gehäuse relativ zu diesem axialverschiebbar gelagert und mittels Federeinrichtungen gegen den jeweiligen Mitnehmer abgestützt. Ferner gehört zu dem Ratschenaufsatz ein Antriebswerkzeug, welches drehbar durch eine Vielecköffnung des Mitnehmers hindurchführbar und mit seinem Kupplungsende drehfest in ein Vieleckprofil des Antriebsrings einrastbar ist. Der Antriebsring bzw. der jeweilige Antriebsring ist mit Hilfe des Antriebswerkzeugs axial von demjenigen Mitnehmer, durch welchen das Antriebswerkzeug hindurchführbar ist, in einer von diesem entkoppelte Stellung wegschiebbar und gleichzeitig in eine gekoppelte Stellung mit dem anderen Mitnehmer, der das Schraubwerkzeug trägt, verschiebbar.

Aus dem Stand der Technik ist etwa eine elektrische, kabelgebundene Schraubpistole bekannt, nämlich aus der US 8 786 233 B2, die zur selben Familie, wie die EP 2 701 879 A1 gehört. Darin wird eine elektrische Ratsche für einen angetriebenen Schraubendreher offenbart. Diese bekannte Ratsche ist derart aufgebaut, dass wenn ein Gasdrücker nicht betätigt ist, dann wird die elektronische Ratsche aktiviert. An einer zusätzlichen Bedieneinheit kann der Anwender zwischen einem Links- und Rechtslauf, sowie einer Blockierung in beide Richtungen wählen.

Die Ratsche ist elektrisch ausgestaltet/aktivierbar, was dazu führt, dass, um die Blockierung der Ratsche zu gewährleisten, ein solch hoher Strom in der Maschine fließt, dass eine starke Hitzeentwicklung zu beklagen ist. Auch fehlt ein akustisches Feedback. Ein, beispielsweise für das Durchführen des Bohrens in der letzten Bohrphase vorgenommenes manuelles Ratschen, oder ein während eines Gewindeeinschneidens verwendetes mechanisches Ratschen, bspw. wie es auch in der letzten Phase des Schraubens eingesetzt ist, ist hier nicht vorhanden. Durch die Kabelgebundenheit wird auch die Reichweite/Bewegungsfreiheit stark eingeschränkt. Ferner ist bei diesem System zu beklagen, dass ein abgeschlossenes System vorgestellt wird und ein versehentliches Betätigen des Gasdrückers während des manuellen Eindrehens zu einem plötzlichen Eindringen der Schraube führt oder zu einem plötzlichen Weiterbohren führt. Dies ist jedoch im Patienteneinsatz verheerend. Auch sind von anderen Herstellern Schraubenaufsätze bekannt, bspw. die "Synthes Bohrpistole 510.01". Auf diese Bohrpistole kann ein Aufsatz aufgesetzt werden, der die Ausgangsdrehzahl der Bohrpistole auf ca. 300 U/min untersetzt. Zur Verwendung ist dabei zusätzlich das Aufsetzen eines Drehmomentbegrenzers auf diesen Schraubenaufsatz vorgeschrieben. Leider sind solche Drehmomentbegrenzer nicht verstellbar und nur in vordefinierten Werten von 0,4 Nm, 0,8 Nm, 1,5 Nm und 4 Nm leicht erhältlich. Ferner ist dieses System nicht zum manuellen Eindrehen und Anziehen von Schrauben verwendbar. Ein Werkzeugwechsel ist daher immer notwendig. Auch zeigt sich in der Praxis die Drehmomentbegrenzung als unflexibel. Auch ein manuelles Eindrehen einer Schraube mit einer mechanischen Ratsche, bspw. mittels eines Schraubendrehers mit integrierter Ratsche, ist nicht zielführend, obwohl hier der Vorteil des Vermeidens des Umgreifens während des Eindrehvorganges vorliegt. Leider ist trotz Ratscheneinsatz immer noch eine solch repetitive Bewegung notwendig, die immer noch zur Ermüdung der Muskulatur führt, also auch das Operationsergebnis unmittelbar negativ beeinflussen kann. Auch die gesundheitlichen Beschwerden der Ärzte werden nicht vermieden.

Ferner ist bei den unterschiedlichen Systemen auch zu beklagen, dass sie entweder nur für Plattenverschraubungen einsetzbar sind oder nur bei Polyaxialschrauben. Polyaxialschrauben sind dabei bspw. solche Schrauben, die einen sphärischen Schraubenkopf aufweisen, der von einem Gehäuse umschlossen ist, derart, dass das Gehäuse frei zur Längsachse der Schraube verstellbar ist. Insbesondere Pedikelschrauben werden so ausgebildet.

Es ist daher die Aufgabe der vorliegenden Erfindung, ein sowohl bei Pedikelschrauben/Polyaxialschrauben als auch bei Plattenverschraubungen einsetzbaren Werkzeugaufnahmeaufsatz zur Verfügung zu stellen, der die vorstehend beschriebenen Nachteile beseitigt oder diese zumindest mildert. Insbesondere soll auch eine Reduktion der körperlichen Belastung des Anwenders (Chirurgen) beim Einbringen von Schrauben erreicht werden. Ferner soll insbesondere die Erfindung dem Anwender (Chirurgen) ermöglichen, dass ein sicheres maschinelles Eindrehen erfolgt. Auch soll die Einstellung der Motordrehzahl so wählbar sein, dass der Anwender die Wunschdrehzahl präzise einstellen kann.

Dies Aufgabe wird durch eine Werkzeugaufnahme mit den Merkmalen des Anspruchs 1 und durch eine Bohrmaschine mit den Merkmalen des Anspruchs 9 erzielt.

Der Grundgedanke der vorliegenden Erfindung beruht darauf, dass zwischen den beiden Kupplungen zum Anschluss eines Motors an der einen Eingangskupplung und zum Aufnehmen/Anschließen eines Werkzeugs an der anderen Ausgangskupplung eine manuell betätigbare / handkraftbetätigbare Antriebseinheit in Form einer Ratsche eingebunden ist, die mittels der Ratschenhandhabe/manuelle Betätigungseinrichtung der Ratsche selbst in eine erste Betriebsposition bringbar ist, in welcher das Drehmoment von der Eingangskupplung unter Umgehung der Ratschenfunktion auf die Ausgangskupplung übertragen wird und in eine zweite Betriebsposition bringbar ist, in welcher die beiden Kupplungen Drehmoment-getrennt sind und stattdessen ein über die Ratschenfunktion von der Ratschenhandhabe/manuelle Betätigungseinrichtung eingetragenes manuelles Drehmoment auf die Ausgangskupplung übertragen wird. Statt einer elektromotorischen Drehmomentweitergabe kann dann eine feiner justierbare manuelle Drehmomentweitergabe bewirkt werden. Dies kann geschehen, ohne dass ein Werkzeugwechsel durchgeführt werden muss und ohne dass die Maschine selbst separat zu der Ratsche in irgendeiner Weise betätigt werden muss. D.h. die vorstehend genannten Betriebspositionen sind ausschließlich durch Ratscheninterne Funktionen und Mittel (Ratschenhandhabe) einstellbar und nicht durch miteinander kommunizierende Funktionen der Ratsche und der Maschine. Somit ist gewährleistet, dass die beiden genannten Betriebspositionen eingestellt und auch gehalten werden können unabhängig davon, in welcher Weise die Maschine betätigt wird, etwa Betätigen des Motors, Aufdrücken des Werkzeugs auf den zu bohrenden Knochen/einzuschraubenden Schraube über die Maschine/den Maschinenhaltegriff, etc.. Auch ist ein somit sicherer Betrieb möglich. Schädigungen des Patienten oder des Arztes werden verhindert. Ein präziser Einsatz wird möglich.

Im Konkreten kann es konstruktiv vorgesehen sein, die Ratsche mit zwei Drehmoment-Übertragungselementen (Hülsen) auszubilden, die jeweils eine gegenläufig wirkende Verzahnung tragen und je nach manueller Auswahl wechselweise in Funktion geschaltet werden können, um so eine rechts- oder linkswirkende Drehmomentübertragung von einem manuellen Eingangsbauteil etwa Ratschengehäuse auf ein Ratschen-Ausgangselement wie Abtriebshülse zu übertragen. Zwischen dem Ratschen-Ausgangselement und den beiden Drehmoment-Übertragungselementen ist eine Art Auswahlbauteil wie Wahlscheibe/-hülse angeordnet, das manuell so verstellt werden kann, dass es mit jeweils einem der Drehmoment-Übertragungselemente in Wirkeingriff kommt. Des Weiteren kann das Auswahlbauteil über das Ratschen-Ausgangselement bei dessen Betätigung mittels der Ratschen-Handhabe in eine Ratschen-Außerfunktionsposition verstellt werden, in welcher die Ratsche kein manuell eingebrachtes Drehmoment auf das Ratschen-Ausgangselement mehr übertragen kann, gleichzeitig aber das Ratschen-Ausgangselement in dieser Betätigungsposition die beiden Kupplungen unmittelbar drehmoment-verbindet.

Beispielsweise ist das Ratschen-Ausgangselement eine Hülse, welche eine Abtriebswelle der Werkzeugaufnahme axialverschiebbar aber drehfest umgreift und eine Verzahnung wie z.B. Stirnverzahnung hat, die in Abhängigkeit der Axialstellung des Ratschen-Ausgangselements bezüglich der Abtriebswelle der Werkzeugaufnahme mit einem direkten/indirekten Abtriebselement der Eingangskupplung in Eingriff bringbar ist. Ferner hat das Ratschen-Ausgangselement eine Art Mitnahme wie z.B. Mitnahmestift oder Vorsprung, die auf das vorstehend genannte Auswahlbauteil der Ratsche wirkt und dieses bei einer Verstellung des Ratschen-Ausgangselements in dessen Ratschen-Außerfunktionsposition in eine Position mitnimmt, in der ein Wirkeingriff zwischen dem Auswahlbauteil und den zwei Drehmoment-Übertragungselementen der Ratsche ausgeschlossen ist. In diesem Fall ist eine Drehmomentübertragung vom Motor auf die Ausgangskupplung ermöglicht, die Ratschenfunktion aber außer Kraft gesetzt. Wird hingegen das Ratschen-Ausgangselements in eine Position verstellt, in welcher kein direkter Wirkeingriff mit dem direkten/indirekten Abtriebselement der Eingangskupplung besteht, wird das Auswahlbauteil in eine Position, z.B. federelastisch gedrängt in der es wahlweise mit einem der zwei Drehmoment-Übertragungselemente in Wirkeingriff bringbar ist. In diesem Fall ist eine Drehmomentübertragung vom Motor auf die Ausgangskupplung unterbrochen, die Ratschenfunktion aber in Kraft gesetzt.

Um die Handhabung der Ratsche zu vereinfachen, kann die Handhabe beispielsweise ein Hebel sein, der am Gehäuse der Ratsche gelagert ist, um dieses um die Abtriebswelle der Werkzeugaufnahme zu drehen und so ein manuelles Drehmoment in die Ratsche einzutragen. Gleichzeitig kann der Hebel in Längsrichtung der Abtriebswelle der Werkzeugaufnahme verschwenkbar am Ratschengehäuse gelagert sein und an seinem in das Ratscheninnere vorragenden Ende auf das Ratschen-Ausgangselement einwirken, um dieses axial zu bewegen und so die beiden vorstehend genannten Positionen für ein außer/in Kraft setzen der Ratschenfunktion einzustellen.

In diesem Fall muss nur die eine, Ratschen-eigene Handhabe betätigt werden, um die beiden Positionen einzustellen und auch ein manuelles Drehmoment in die Ratsche einzutragen.

Vorteilhafte Ausführungsformen sind auch in den Unteransprüchen beansprucht und werden nachfolgend näher erläutert.

So ist es von Vorteil, wenn die zum Drehmoment bereitstellen per Handkraft eingerichtete Antriebseinheit (Gehäuse) mit einer Ratscheinheit / Ratscheinrichtung oder einer Knarre (innerhalb des Gehäuses) verbunden ist oder mit ihr integriert ist. Eine umlaufende Drehbewegung innerhalb eines nur begrenzten Arbeitsraumes, z.B. zum Lösen oder Festziehen von Schraubverbindungen, wird dann effizient ermöglicht. Dazu kann eine Zahnung im Inneren der Ratscheinheit / Ratscheinrichtung oder Knarre eingesetzt werden, bspw. einen Drehwinkel von wenigstens 10° bis 15° erfordernd, um eine Drehung eines Abtriebselements zu erreichen. Es können aber auch Feinzahnratschen eingesetzt werden, so dass bereits ein Drehwinkel von rund 5° genügt, um eine Bewegung der Schraube zu zeitigen.

Es ist auch von Vorteil, wenn die Ratsche oder Knarre der manuellen Antriebseinheit (Ratschen-Eingangselement/Ratschengehäuse) nachgeschaltet ist, d.h. zwischen der manuellen Antriebseinheit und der abtriebsseitigen Kupplung (Ausgangskupplung) eingesetzt ist. Auf diese Weise kann ein effizientes Funktionieren erreicht werden.

Um eine akustische Rückkopplung zu erleichtern, ist es von Vorteil, wenn die Ratscheinheit / Ratscheinrichtung als mechanische Ratsche ausgestaltet ist. Zusätzlich ist es möglich, über eine Richtungswahlbedieneinheit zwischen einem Rechtslauf und einem Linkslauf hin- und her zu schalten. Auf diese Weise können Schaltstellungen zum Einschrauben oder stattdessen zum Ausschrauben vorgehalten werden.

Um eine Fehlbedienung zu vermeiden, ist es von Vorteil, wenn die Richtungswahlbedieneinheit so eingebunden ist, dass eine Betätigung nur im elektrischen Betrieb möglich ist.

Auch ist es von Vorteil, wenn die manuelle Antriebseinheit mit dem händisch greifbaren Hebel verbunden ist. Der Hebel kann dann Z-, N- oder S-förmig ausgestaltet sein. Wenn eine Drehmomentbegrenzungseinrichtung eingebunden ist, kann verhindert werden, dass über ein Grenzdrehmoment hinaus gebohrt oder geschraubt wird. Die Sicherheit in der Bedienung wird erhöht.

Ein vorteilhaftes Ausführungsbeispiel ist auch dadurch gekennzeichnet, dass die Drehmomentbegrenzungseinrichtung zwischen der antriebsseitigen Kupplung und der Ratscheinrichtung / Ratscheinheit angeordnet ist.

Es ist zweckmäßig, wenn die manuelle Antriebseinheit mit einer Trennkupplung derart verbunden ist, dass im Fall einer Betätigung der manuellen Antriebseinheit eine motorische Drehmomentweitergabe an die abtriebsseitige Kupplung ausgeschlossen wird. Vorteilhaft ist es, die Trennkupplung zu jedem Zeitpunkt betätigen zu können. Ein fließender oder digitaler Wechsel zwischen dem elektrischen und manuellen Betriebsmodus wird dadurch ermöglicht.

Hierbei ist es von Vorteil, wenn die Trennkupplung mit dem Hebel der manuellen Antriebseinheit derart verbunden ist, dass bei einem entsprechenden Betätigen des Hebels der Ratsche die Trennkupplung betätigt wird.

Die Erfindung betrifft auch eine chirurgische Bohrmaschine bzw. einen chirurgischen Schraubendreher mit einem Elektromotor, der über die antriebsseitige Kupplung des erfindungsgemäßen Werkzeugaufnahmeaufsatzes zum Drehmomenteinleiten in diesen Aufsatz verbunden ist.

Mit anderen Worten betrifft die Erfindung also einen Werkzeugaufnahmeaufsatz, bei dem sich durch einen manuellen Betriebsmodus der Anwender zu jedem Zeitpunkt entscheiden kann, ohne das Werkzeug zu wechseln, die Schraube manuell einzudrehen. Er erhält dadurch ein direkteres Feedback über den Zustand des Knochens. Durch eine mechanische Ratsche mit umschaltbarem Links- und Rechtslauf, wird dieses manuelle Eindrehen komfortabler gemacht. Um ein versehentliches Umschalten während des Eindrehens zu verhindern, kann die Drehrichtung (Links- / Rechtslauf) nur im elektrischen Modus gewechselt werden.

Eine frei einstellbare Drehmomentbegrenzung, welche nur im elektrischen Modus wirksam ist, unterstützt den Anwender bei der Wahl des richtigen Zeitpunktes, ab dem er manuell eindrehen möchte. Sie kann zusätzlich als Sicherheitsfunktion verwendet werden. Durch die freie Einstellbarkeit ist eine flexible Anpassung in verschiedene Einsatzbereiche möglich. Ein versehentliches Verstellen wird durch einen Verriegelungsmechanismus verhindert.

Es wird eine mechanische Umsetzung eines manuellen Antriebs auf einer chirurgischen Bohrmaschine ermöglicht. Genauer, wird eine Umsetzung eines manuellen Antriebs in einem Aufsatz für eine chirurgische Bohrmaschine erreicht. Eine zusätzliche Integration einer Ratschfunktion wird erreicht. Ein Wechsel der Drehrichtung der Ratschfunktion ist vorgesehen. Ein fließender oder digitaler Wechsel zwischen einem elektrischen und manuellen Betriebsmodus wird durch einen Hebel ermöglicht. Die zusätzliche Kombination mit einer frei einstellbaren Drehmomentbegrenzung, die rastet oder stufenlos sein kann, aber auch auf eine Nm-Skala verzichten kann, ist von Vorteil. Die Umsetzung dieser Funktion ist in einem einzigen Aufsatz möglich.

Es wird ein elektrisches und manuelles Schraubeneindrehen ohne Werkzeugwechsel ermöglicht. Die körperliche Belastung des Chirurgen wird gesenkt. Die Bediensicherheit wird erhöht, da ein Betätigen eines "Gasdrückers" im manuellen Modus unkritisch ist. Die Ratsche im manuellen Modus erhöht den Komfort beim manuellen Eindrehen. Ein akustisches Feedback wird durch die Ratsche, die ein Ratsch-Geräusch hervorruft, ermöglicht. Die Sicherheit wird durch eine einstellbare Drehmomentbegrenzung erhöht.

Ein akustisches Feedback beim Durchrutschen der Drehmomentbegrenzung wird ebenfalls erzwungen. Mit einer passenden Kupplung ist der Werkzeugaufnahmeaufsatz an jeder Antriebsmaschine einsetzbar, und zwar rein mechanisch. Es wird keine Elektronik benötigt, welche Probleme bei der Sterilisierbarkeit haben könnte. Eine einfache Bedienung ist die Folge.

Das manuelle Eindrehen von Schrauben erfordert einen hohen Zeit- und Kraftaufwand. Gleichzeitig können Ärzte nicht rein maschinell eindrehen, da das taktile Feedback fehlt. Die Erfindung macht es ohne Werkzeugwechsel möglich, Schrauben nach Wahl manuell und maschinell einzudrehen. Zur Unterstützung kann zusätzlich ein Begrenzungsdrehmoment frei eingestellt werden.

Der Aufsatz wird über die "Plug-and-Play"-Kopplung an der Antriebsmaschine angekoppelt. Am distalen Ende befindet sich eine weitere "Plug-and-Play"-Kupplung, die in der Lage ist, ein Schraubwerkzeug aufzunehmen. Durch Entriegeln und Verstellen des Drehgriffs kann das Ausrastdrehmoment bei elektrischem Antrieb eingestellt werden. Nach Einstellung des Drehmoments kann der Drehgriff verriegelt werden. Um den Anwender bei der Anwendung zu unterstützen, kann der Drehgriff gerastert sein.

Durch ein Ziehen des Hebels in Längsrichtung der Werkzeugaufnahmewelle, wie dies vorstehend bereits angedeutet wurde, kann der Anwender Schrauben manuell in den Knochen über die Ratsche eindrehen. Im Umsetzungsbeispiel wird der Abtrieb derart komplett vom elektrischen Antrieb (Motor) getrennt, was die Bediensicherheit zusätzlich erhöht. Bei Loslassen des Hebels wird die Verbindung mit dem elektrischen Antrieb wieder hergestellt. Der manuelle Antrieb ist als verstellbare Ratsche umgesetzt. Mit einem weiteren Bedienelement kann der Anwender zwischen Links- und Rechtslauf wechseln. Um ein versehentliches Verstellen zu verhindern, ist die Betätigung nur im elektrischen Betriebsmodus möglich. Bei der Gestaltung des Bedienelementes wird Wert auf eine intuitive Bedienung gelegt. Folglich entspricht dem Umsetzungsbeispiel die vordere Position des Bedienelementes dem Rechtslauf, also dem Eindringen einer Schraube.

Die Erfindung wird nachfolgend mit Hilfe einer Zeichnung näher erläutert. Dabei ist ein erstes Ausführungsbeispiel dargestellt. Es zeigen:
- Fig. 1: eine Seitenansicht eines erfindungsgemäßen Werkzeugaufnahmeaufsatzes,
- Fig. 2: eine erfindungsgemäße chirurgische Bohrmaschine, die auch als chirurgischer Schraubendreher verwendet werden kann, mit einem erfindungsgemäß adaptierten Werkzeugaufnahmeaufsatz in einer Seitenansicht, und
- Fig. 3: eine Längsschnittansicht durch eine erfindungsgemäße Bohrmaschine.

Die Figuren dienen nur dem Verständnis der Erfindung. Gleiche Elemente sind mit denselben Bezugszeichen versehen.

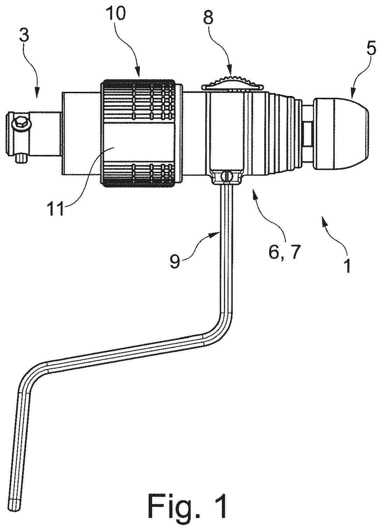

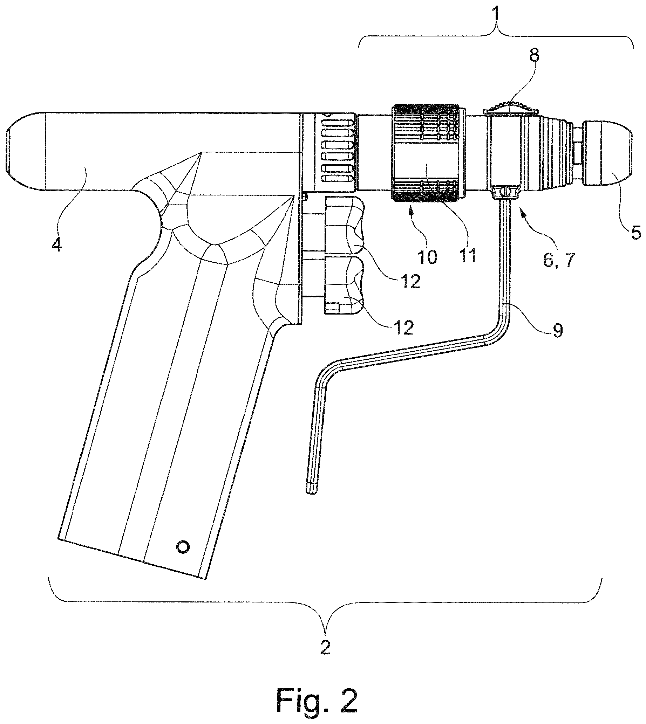

In Fig. 1 ist ein erfindungsgemäßer Werkzeugaufnahmeaufsatz 1 dargestellt. Er ist für die in der Fig. 2 dargestellte chirurgische Bohrmaschine 2 vorgesehen und dort angekoppelt.

Zurückkommend auf Fig. 1 sei erläutert, dass der Werkzeugaufnahmeaufsatz 1 eine antriebsseitige Kupplung (Eingangskupplung) 3 zum Anbringen eines Antriebsaggregates 4 (siehe Fig. 2) wie Motor, Motor-Getriebe-Einheit, etc. aufweist. Die antriebsseitige Kupplung 3 ist als "Plug-and-Play-Kupplung" ausgestaltet. Sie ist also eine rekonfigurationslose Kupplung oder eine Kupplung, die frei von einer benutzerabhängigen Nachjustierung ist. Das Antriebsaggregat kann eine medizinische/chirurgische Maschine wie Bohr-/Schraub-/Fräsmaschine sein, wie sie aus dem Stand der Technik hinlänglich bekannt ist und daher an dieser Stelle nicht im Einzelnen beschrieben werden muss.

Während am proximalen Ende des Werkzeugaufnahmeaufsatzes 1 die antriebsseitige Kupplung 3 vorhanden ist, ist am distalen Ende eine abtriebsseitige Kupplung (Ausgangskupplung) 5 vorhanden.

Zwischen den beiden Kupplungen 3 und 5, wobei auch die abtriebsseitige Kupplung 5 als "Plug-and-Play-Kupplung" ausgestaltet ist, ist eine eigene, separate manuell betätigbare, d.h. über Handkraft betätigbare Antriebseinheit 6 vorhanden. Die Antriebseinheit 6 ist mit einer Ratscheinrichtung / Ratscheinheit 7 verbunden / integriert, wobei die Ratscheinrichtung / Ratscheinheit 7 als manuelle Ratsche ausgebildet ist.

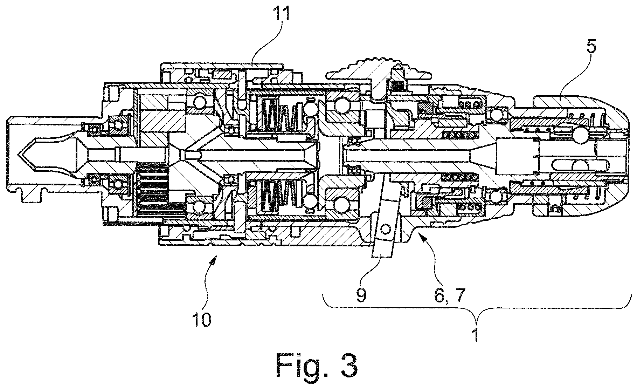

Im Konkreten besteht die Ratsche 7 gemäß der Fig. 3 aus einem Gehäuse als die manuelle Antriebseinheit 6, in welchem zwei hülsenförmige Drehmoment-Übertragungselemente drehfest mit dem Gehäuse 6 aufgenommen sind, von denen zumindest ein Drehmoment-Übertragungselement axialverschiebbar bezüglich des anderen Drehmoment-Übertragungselements gehalten ist. Beide Drehmoment-Übertragungselemente weisen vorzugsweise an deren eine Stirnseite jeweils eine Verzahnung auf, um wechselweise sowie wahlweise in Abhängigkeit ihrer axialen Relativlage zueinander mit einem Zwischen-/Auswahlbauteil in Form einer axial zu den Drehmoment-Übertragungselementen benachbarten Scheibe oder eines Rings in Kämmeingriff gebracht zu werden.

Diese relative Axialposition lässt sich mittels eines manuell betätigbaren Schiebers einstellen. Der Schieber ist dabei mit einem der beiden Drehmoment-Übertragungselemente gekoppelt, um dieses axial zu verschieben. Dadurch wird das zumindest eine axial verschiebbare Drehmoment-Übertragungselement mit dem Zwischen-/Auswahlbauteil in oder außer Kämmeingriff gebracht. Das Zwischen-/Auswahlbauteil ist ebenfalls axialverschieblich gehalten und mittels einer Feder in Richtung hin zu den beiden Zwischen-/Auswahlbauteilen vorgespannt. Wird somit das eine Drehmoment-Übertragungselement mittels des Schiebers gegen das Zwischen-/Auswahlbauteil axial in Kämmeingriff gedrückt, wird dieses gegen die Federvorspannung axial verschoben, wodurch der Kämmeingriff mit dem anderen Drehmoment-Übertragungselement aufgehoben wird und umgekehrt.

Die Verzahnungen der beiden Drehmoment-Übertragungselemente wirken dabei gegenläufig zueinander, derart, dass sie einen Rechts- oder Links-Drehmoment übertragen können und in die jeweils andere Richtung einen Freilauf bewirken. Je nach ausgewähltem Drehmoment-Übertragungselement kann so ein Rechts- oder Linkswirkendes Drehmoment ausgehend von der manuellen Antriebseinheit 6 über die Ratsche übertragen werden. Das Zwischen-/Auswahlbauteil ist ferner axialverschieblich aber drehfest auf einem hülsenförmigen Ratschen-Ausgangselement gelagert, das wiederum drehfest aber axialverschieblich auf einer Welle der abtriebsseitigen Kupplung 5 gelagert ist.

Das Ratschen-Ausgangselement hat vorzugsweise stirnseitig eine Verzahnung, über die das Ratschen-Ausgangselement in Abhängigkeit seiner Axialposition bezüglich der Welle der abtriebsseitigen Kupplung 5 mit einem Ausgangselement der eingangsseitigen Kupplung 3 in drehmoment-übertragenden Kämmeingriff kommen kann, um ein Drehmoment vom Motor auf die ausgangsseitige Kupplung 5 zu übertragen.

Die manuelle Antriebseinheit (Gehäuse) 6 wird über einen Hebel 9 aktiviert und angetrieben. Dafür ist der Hebel 9 nach Art einer Kurbel ausgebildet und an die Größe einer menschlichen Hand angepasst. D.h. der Hebel 9 ist im Gehäuse 6 derart gelagert, das das Gehäuse 6 über diesen um die Mittelachse der Werkzeugaufnahme gedreht und damit die Drehmoment-Übertragungselemente angetrieben werden können. Der Hebel 9 ist aber in Axialrichtung der Werkzeugaufnahme schwenkbar gelagert, wie dies in der Fig. 3 gezeigt ist. Ein in das Gehäuseinnere vorragender Hebelfortsatz ist mit dem Ratschen-Ausgangselement so gekoppelt, dass dieses durch ein Verschwenken des Hebels 9 in Axialrichtung verschoben werden kann.

Das Ratschen-Ausgangselement hat eine Mitnahme in Form eines Radialvorsprungs, der direkt oder indirekt auf das Zwischen-/Auswahlbauteil der Ratsche einwirkt, um dieses entsprechend der Axialbewegung des Ratschen-Ausgangselements ggf. mitzunehmen.

Wird demnach das Ratschen-Ausgangselement über den Hebel 9 axial gegen das Ausgangselement der eingangsseitigen Kupplung 3 in drehmoment-übertragenden Kämmeingriff verschoben, wird das Zwischen-/Auswahlbauteil der Ratsche in eine Axialposition mitgenommen, in der es nicht mehr in Kämmeingriff mit einem der zwei Drehmoment-Übertragungselemente kommen kann. In diesem Fall wird zwar ein Drehmoment vom Motor auf die ausgangsseitige Kupplung 5 übertragen, die Ratschenfunktion wird aber außer Kraft gesetzt. Wird hingegen das Ratschen-Ausgangselement über den Hebel 9 axial vom Ausgangselement der eingangsseitigen Kupplung 3 weg verschoben (kein Kämmeingriff mehr), wird das Zwischen-/Auswahlbauteil der Ratsche in eine Axialposition mitgenommen, in der es in Kämmeingriff mit einem der zwei Drehmoment-Übertragungselemente kommen kann. In diesem Fall kann zwar kein Drehmoment vom Motor auf die ausgangsseitige Kupplung 5 übertragen werden, die Ratschenfunktion wird aber in Kraft gesetzt. Dadurch wird quasi eine Trennkupplung realisiert.

Auf diese Weise erhält der Hebel 9 zwei Funktionen, nämlich
- das in/außer Kraft setzen der Ratschenfunktion bei gleichzeitigem An-/Abkoppeln des Motors mit/von der abtriebsseitigen Kupplung 5 und
- das manuelle Betätigen der Ratsche.

Zwischen der manuellen Ratsche insbesondere zwischen dem Ratschen-Ausgangselement und der antriebsseitigen Kupplung 3 ist auch eine Drehmomentbegrenzungseinrichtung 10 vorhanden. Diese ist manuell einstellbar, entriegel- und verriegelbar, bspw. über einen Drehgriff. Der Drehgriff ist mit dem Bezugszeichen 11 versehen.

In Fig. 2 und Fig. 3 ist der komplette Aufbau der chirurgischen Bohrmaschine 2 mit eingesetztem Werkzeugaufnahmeaufsatz 1 dargestellt. Dabei sind zwei Betätigungsknöpfe 12 eingesetzt, mit denen unterschiedliche Funktionen der Bohrmaschine 2 gesteuert / geregelt werden können.

### Bezugszeichenliste

- 1: Werkzeugaufnahmeaufsatz
- 2: chirurgische Bohrmaschine
- 3: antriebsseitige Kupplung
- 4: Antriebsaggregat
- 5: abtriebsseitige Kupplung
- 6: manuelle Antriebseinheit / Gehäuse

- 7: Ratscheinrichtung / Ratscheinheit
- 8: Richtungswahlbedieneinheit
- 9: Hebel
- 10: Drehmomentbegrenzungseinrichtung
- 11: Drehgriff
- 12: Betätigungsknopf

## Patentansprüche

1. Werkzeugaufnahmeaufsatz (1) für eine chirurgische Maschine, vorzugsweise Bohrmaschine (2) mit einer antriebsseitigen Kupplung (3) zum Anbringen an einem motorisch Drehmoment bereitstellenden Antriebsaggregat (4), mit einer abtriebsseitigen Kupplung (5) zum Aufnehmen eines Werkzeugs, wobei zwischen den beiden Kupplungen (3, 5) eine manuell betätigbare Antriebseinheit (6) in Form einer Ratscheneinheit (7) eingebunden ist, **dadurch gekennzeichnet, dass** die Ratscheneinheit (7) eine Handhabe (9) aufweist, mittels der die Ratscheneinheit (7) in eine erste Betriebsposition bringbar ist, in welcher das Drehmoment von der antriebsseitigen Kupplung (3) unter Umgehung der Ratscheneinheit (7) auf die abtriebsseitige Kupplung (5) übertragbar ist und in eine zweite Betriebsposition bringbar ist, in welcher die antriebs- und abtriebsseitige Kupplung (3, 5) Drehmoment-getrennt sind und ein über die Ratscheneinheit (7) eingetragenes manuelles Drehmoment auf die abtriebsseitige Kupplung (5) übertragbar ist.

2. Werkzeugaufnahmeaufsatz (1) nach Anspruch 1, **dadurch gekennzeichnet, dass** die Antriebseinheit (6) dazu eingerichtet ist, Drehmoment per Handkraft bereitzustellen, und mit der Ratscheneinheit (7) integriert ist.

3. Werkzeugaufnahmeaufsatz (1) nach Anspruch 1 oder 2, **dadurch gekennzeichnet, dass** die Ratscheneinheit (7) als mechanische Ratsche ausgestaltet ist und über eine Richtungswahlbedieneinheit (8) zwischen einem Rechtslauf und einem Linkslauf hin- und herschaltbar ist.

4. Werkzeugaufnahmeaufsatz (1) nach einem der Ansprüche 2 bis 3, **dadurch gekennzeichnet, dass** die manuelle Antriebseinheit (6) mit der Handhabe in Form eines händisch greifbaren Hebels (9) verbunden ist.

5. Werkzeugaufnahmeaufsatz (1) nach einem der vorgehenden Ansprüche, **dadurch gekennzeichnet, dass** eine Drehmomentbegrenzungseinrichtung (10) eingebunden ist.

6. Werkzeugaufnahmeaufsatz (1) nach Anspruch 5, **dadurch gekennzeichnet, dass** die Drehmomentbegrenzungseinrichtung (10) zwischen der abtriebsseitigen Kupplung (5) und der Ratscheneinheit (7) angeordnet ist.

7. Werkzeugaufnahmeaufsatz (1) nach einem der Ansprüche 2 bis 4, **dadurch gekennzeichnet, dass** er eine Trennkupplung aufweist, und dass die manuelle Antriebseinheit (6) mit der Trennkupplung derart verbunden ist, dass bei Betätigung der manuellen Antriebseinheit (6) eine motorische Drehmomentweitergabe an die abtriebsseitige Kupplung (5) verunmöglicht ist.

8. Werkzeugaufnahmeaufsatz (1) nach Anspruch 7, **dadurch gekennzeichnet, dass** die Trennkupplung mit der Handhabe (9) der manuellen Antriebseinheit (6) derart verbunden ist, dass bei Betätigung des Hebels (9) die Trennkupplung betätigt wird.

9. Chirurgische Bohrmaschine (2) aufweisend einen Werkzeugaufnahmeaufsatz (1) nach einem der vorhergehenden Ansprüche.

## Claims

1. A tool fitting attachment (1) for a surgical machine, preferably a drill (2), comprising a drive-side coupling (3) for mounting to a drive unit (4) which provides a torque by means of a motor, and comprising an output-side coupling (5) for receiving a tool, wherein between the two couplings (3, 5), a manually operated drive unit (6) is integrated in the form of a ratchet unit (7), **characterized in that** the ratchet unit (7) has a handle (9) by means of which the ratchet unit (7) can be brought into a first operating position, in which the torque is transmittable from the drive-side coupling (3) to the output-side coupling (5), bypassing the ratchet unit (7), and can be brought into a second operating position, in which the drive-side and output-side couplings (3, 5) are torque-separated and a manual torque entered via the ratchet unit (7) is transmittable to the output-side coupling (5).

2. The tool fitting attachment (1) according to claim 1, **characterized in that** the drive unit (6) is configured to provide a torque by manual force and is integrated with the ratchet unit (7).

3. The tool fitting attachment (1) according to claim 1 or 2, **characterized in that** the ratchet unit (7) is in the form of a mechanical ratchet and is changeable between a clockwise rotation and an anti-clockwise rotation via a direction selection control unit (8).

4. The tool fitting attachment (1) according to one of claims 2 to 3, **characterized in that** the manual drive unit (6) is connected to the handle in the form of a manually graspable lever (9).

5. The tool fitting attachment (1) according to one of the preceding claims, **characterized in that** a torque limiter (10) is integrated.

6. The tool fitting attachment (1) according to claim 5, **characterized in that** the torque limiter (10) is arranged between the output-side coupling (5) and the ratchet unit (7).

7. The tool fitting attachment (1) according to one of the claims 2 to 4, **characterized in that** it comprises a disconnect coupling, and that the manual drive unit (6) is connected to the disconnect coupling so that upon actuation of the manual drive unit (6), a motor-driven torque transmission to the output-side coupling (5) is rendered impossible.

8. The tool fitting attachment (1) according to claim 7, **characterized in that** the disconnect coupling is connected to the handle (9) of the manual drive unit (6) so that upon actuation of the lever (9) the disconnect coupling is actuated.

9. A surgical drill (2) comprising a tool fitting attachment (1) according to one of the preceding claims.

## Revendications

1. Embout de logement d'outil (1) pour une machine chirurgicale, de préférence perceuse (2) avec un accouplement (3) côté entraînement à monter sur un groupe d'entraînement (4) fournissant un couple de manière motorisée, avec un accouplement (5) côté sortie pour recevoir un outil, dans lequel une unité d'entraînement (6) pouvant être actionnée manuellement sous forme d'une unité à cliquet (7) est intégrée entre les deux accouplements (3, 5), **caractérisé en ce que** l'unité à cliquet (7) présente une poignée (9), au moyen de laquelle l'unité à cliquet (7) peut être amenée dans une première position de fonctionnement, dans laquelle le couple peut être transmis de l'accouplement (3) côté entraînement à l'accouplement (5) côté sortie avec contournement de l'unité à cliquet (7) et peut être amené dans une deuxième position de fonctionnement, dans laquelle les accouplements (3, 5) côté entraînement et sortie sont séparés du couple et un couple manuel introduit par l'intermédiaire de l'unité à cliquet (7) peut être transmis à l'accouplement (5) côté sortie.

2. Embout de logement d'outil (1) selon la revendication 1, **caractérisé en ce que** l'unité d'entraînement (6) est conçue pour fournir un couple par force manuelle, et est intégrée avec l'unité à cliquet (7).

3. Embout de logement d'outil (1) selon la revendication 1 ou 2, **caractérisé en ce que** l'unité à cliquet (7) est conçue sous la forme d'un cliquet mécanique et peut être commutée en va-et-vient entre une marche à droite et une marche à gauche par l'intermédiaire d'une unité de commande utilisateur de sélection de direction (8).

4. Embout de logement d'outil (1) selon l'une quelconque des revendications 2 à 3, **caractérisé en ce que** l'unité d'entraînement (6) manuelle est reliée à la poignée sous forme d'un levier (9) pouvant être saisi à la main.

5. Embout de logement d'outil (1) selon l'une quelconque des revendications précédentes,
**caractérisé en ce qu'**un système de limitation de couple (10) est intégré.

6. Embout de logement d'outil (1) selon la revendication 5,
**caractérisé en ce que** le système de limitation de couple (10) est disposé entre l'accouplement (5) côté sortie et l'unité à cliquet (7).

7. Embout de logement d'outil (1) selon l'une quelconque des revendications 2 à 4,
**caractérisé en ce qu'**il présente un embrayage de séparation, et que l'unité d'entraînement (6) manuelle est reliée à l'embrayage de séparation, de telle sorte que lors de l'actionnement de l'unité d'entraînement (6) manuelle un transfert de couple moteur à l'accouplement (5) côté sortie est rendu impossible.

8. Embout de logement d'outil (1) selon la revendication 7, **caractérisé en ce que** l'embrayage de séparation est relié à la poignée (9) de l'unité d'entraînement (6) manuelle, de telle sorte que lors de l'actionnement du levier (9) l'embrayage de séparation est actionné.

9. Perceuse chirurgicale (2) présentant un embout de logement d'outil (1) selon l'une quelconque des revendications précédentes.
